# EUROPÄISCHE PATENTANMELDUNG

(11) **EP 0 808 607 A1**
(43) Veröffentlichungstag der Anmeldung: **26.11.1997**
(21) Anmeldenummer: 97107093.3
(22) Anmeldetag: 29.04.1997
(51) Int. Cl.: A61B 17/39, A61N 1/05

(54) **Vorrichtung zum Perforieren der Herzwand**

(30) Priorität: 24.05.1996 DE 19621099
(71) Anmelder: Sulzer Osypka GmbH, 79639 Grenzach-Wyhlen (DE)
(72) Erfinder: Osypka, Peter Dr., 79639 Grenzach-Wyhlen (DE)
(74) Vertreter: Schmitt, Hans, Dipl.-Ing.

(57) **Zusammenfassung**

Eine Vorrichtung (1) dient zum Anbringen von Perforationen, Lochungen oder Perfusionskanälen in der Herzwand (2), insbesondere im Myokard, zur Erzeugung von in der Herzwand (2) verlaufenden Kanälen für deren Durchblutung. Damit der Brustkorb zum Anbringen solcher Perforationen nicht in einer Operation geöffnet werden muß, um das Herz freizulegen, weist die Vorrichtung (1) einen intrakardial in das Herz (3) einführbaren Katheter (4) auf, der an seinem Arbeitsende wenigstens eine Elektrode oder einen Pol und eine Verbindung dieser Elektrode oder des Poles zu einem Hochfrequenzgenerator (5) hat. Dieses distale Arbeitsende ist dabei wenigstens eine von der Innenseite in die Herzwand (2) einstechbare Nadel (6), die als Koagulationswerkzeug wirkt, so daß das Lumen des von ihr erzeugten Kanales offen bleibt und von dem Inneren des Herzens (3) aus mit frischem Blut versorgt werden kann.

## Beschreibung

Die Erfindung betrifft eine Vorrichtung zum Anbringen von Perforationen, Lochungen oder Kanälen in der Herzwand, insbesondere im Myokard, zur Erzeugung von in der Herzwand verlaufenden Kanälen für deren Durchblutung.

Zur Behandlung von minderdurchbluteten Bereichen des Herzmuskels ist es bekannt, von der Außenseite mit Hilfe von Laserstrahlen Perforationen und Kanäle anzubringen, die nach einer gewissen Zeit an der Herzaußenseite wieder zuwachsen, im Inneren der Herzwand aber aufgrund der Energie des Laserstrahles als bleibende Kanäle bestehen bleiben, die von der Herzinnenseite mit einem Teil des von dem Herzen beförderten Blutes durchblutet werden, so daß auf diese Weise eine Verbesserung der allgemeinen Durchblutung des Herzmuskels erreicht werden kann. Dabei hat sich gezeigt, daß solche durch Laserstrahlen angebrachte Perforationen in der Herzwand sogar kanalartige Seitenäste ausbilden können, so daß es genügt, derartige Perforationen in einem gewissen Abstand anzubringen, um ein relativ großes Areal des Herzmuskels wieder besser zu durchbluten.

Diese Behandlung des Herzens erfordert allerdings eine Öffnung des Brustkorbes (Thorakotomie), was bei Patienten, deren Herz nur noch schlecht durchblutet ist, nicht in allen Fällen möglich ist, weil sie eine derart schwere Operation unter Umständen nicht aushalten. Darüber hinaus blutet das Herz an der frischen Operation eine zeitlang, so daß der Patient auch einen Blutverlust erleidet.

Außerdem kann nur ein relativ kurzer, etwa der Dicke der Herzwand entsprechender Kanal gebildet werden und ob dabei auch Seitenäste in genügender Zahl entstehen, um zu einer weiträumigen Durchblutung zu führen, ist ungewiß. Somit steht der Operateur vor der Frage, wie viele derartige Perforationen in welchen Abständen er anbringen soll, um die Operation zu einem Erfolg zu führen.

Der Erfindung liegt deshalb die Aufgabe zugrunde, eine Vorrichtung zur Anbringung von Peforationen in der Herzwand der eingangs genannten Art zu schaffen, bei der Nachblutungen an der Außenseite und vor allem ein Öffnen des Brustkorbes vermieden werden können.

Die überraschende Lösung dieser widersprüchlichen Aufgabe besteht darin, daß die Vorrichtung einen Katheter mit einem Arbeitsende aufweist, das wenigstens eine Elektrode oder einen Pol und eine Verbindung dieser Elektrode oder des Poles zu einem Hochfrequenzgenerator hat und intrakardial in das Herz einführbar ist, und daß dieses distale Arbeitsende eine von der Innenseite in die Herzwand mehrfach einstechbare gerade, gekrümmte und/oder gewendelte Nadel oder Werkzeug als Hochfrequenzpol oder -elektrode aufweist.

Es kann also zur Anbringung der Perforationen, Lochungen oder Kanäle in der Herzwand für deren bessere Durchblutung von der Herzinnenseite her geareitet werden, indem ein entsprechender Katheter intrakardial durch ein Blutgefäß eingeführt wird, was eine auch von Herzschrittmachern bekannte Technik ist. Somit lassen sich von der Innenseite her mit einer Nadel oder einem entsprechenden Werkzeug durch mehrfaches Einstechen Kanäle in dem Myokard anbringen und so koagulieren, daß sie bestehen bleiben und nicht wieder zuwachsen. Eine Öffnung des Brustkorbes kann also vermieden werden und darüber hinaus kann auch eine vollständige Perforation der Herzwand unterbleiben. Ein weiterer Vorteil besteht darin, daß dieser Katheter mit seinem Werkzeug auch dazu verwendet werden kann, im Inneren des Herzens befindliches störendes Gewebe zu koagulieren (Ablation).

Eine Ausführungsform der Erfindung kann darin bestehen, daß eine monopolare Koagulationsnadel als Arbeitsende oder Werkzeug vorgesehen ist und eine zweite indifferente Elektrode zur außenseitigen Anwendung an einem Patienten mit dem Hochfrequenzgenerator verbindbar ist.

Eine abgewandelte Ausführungsform kann vorsehen, daß eine bipolare Koagulationsnadel als Arbeitsende vorgesehen ist, die zueinander beabstandet und gegeneinander isoliert beide mit dem Hochfrequenzgenerator verbundenen oder verbindbaren Elektroden oder Pole aufweist. Im letzteren Fall kann die außen am Patienten anlegbare indifferente Elektrode vermieden werden, in deren Bereich es unter Umständen zu Verbrennungen kommen kann.

Besonders zweckmäßig ist es, wenn die Nadel oder das Werkzeug in das distale Ende des Katheters einschiebbar oder einziehbar und zum Einstechen in die Innenseite der Herzwand daraus ausschiebbar ist. Somit kann der Katheter zunächst durch ein Blutgefäß in das Herzinnere eingeführt werden, während die Nadel oder das Werkzeug noch zurückgezogen ist, so daß also Verletzungen des Gefäßes durch dieses Werkzeug vermieden werden. Hat der Katheter mit seinem distalen Ende die zu perforierende Stelle im Inneren des Herzens erreicht, kann die etwa in seiner axialen Richtung verschiebbare Nadel in die Herzwand eingeführt werden und zwar so, daß diese nicht völlig durchstochen wird. In dem dabei oder dann erfolgenden Koagulationsvorgang kann dann die Perforation zu einem dauerhaften Kanal gemacht werden.

Besonders günstig ist es zur Erlangung möglichst langer Kanäle innerhalb der Herzwand, also zur Erlangung von Kanälen, die auch länger sein können, als die Herzwand dick ist, wenn die gekrümmte Nadel etwa einen Viertelkreis bis einen Halbkreis oder gegebenenfalls etwas mehr als einen Halbkreis bildet. Eine über etwa einen Halbkreis oder gar darübergehende Krümmung der Nadel kann dabei zu Kanälen führen, die an beiden Enden zum Herzinneren hin offen sind. Es ist aber auch möglich, aufgrund der Biegsamkeit der Nadel oder des Werkzeuges dieses so in die Herzwand einzuführen, daß in Gebrauchsstellung die Krümmung teilweise aufgehoben ist, also das freie Ende der Nadel nicht wieder aus der Herzwand austritt, so daß ein entsprechend noch längerer Kanal entsteht, der von seinem Eintritt von der Innenseite in die Herzwand abgesehen, über eine relativ große Länge im Inneren der Herzwand zwischen deren Innen- und Außenseite verläuft.

Um ein völliges Durchstechen der Herzwand zu vermeiden, kann der Verschiebeweg der Nadel oder des Werkzeuges relativ zu dem distalen Ende des Katheters geringer als die Dicke der Herzwand sein. Es können aber auch andere oder zusätzliche Maßnabmen vorgesehen sein.

Beispielsweise kann die Nadel elastisch und vorgekrümmt sein und eim Ausschieben aus dem distalen Ende des Katheters in ihre gekrümmte Form übergehen. Sie kann dann länger sein, ohne die Herzwand zu durchstechen, weil sie von dem distalen Ende des Katheters aus eine gekrümmte Perforation in der Herzwand anbringt, die schräg und gekrümmt in diese eintritt und aufgrund der Krümmung des Werkzeuges nicht durch die Herzwand hindurchgeht. Dadurch kann gleichzeitig das völlige Durchstechen der Herzwand vermieden und ein längerer Kanal für die Durchblutung geschaffen werden.

Eine weitere vorteilhafte und zweckmäßige Ausgestaltung der Erfindung kann darin bestehen, daß die Koagulationsnadel oder dergleichen Werkzeug relativ zu dem Katheter verdrehbar ist. Die Fortsetzung der Nadel am proximalen Ende des Katheters kann beispielsweise einen Drehgriff aufweisen, so daß der Benutzer die Nadel in eine beliebige Form drehen kann, was in Verbindung mit ihrer Vorkrümmung zweckmäßig ist. Beispielsweise kann eine vorgekrümmte derartige Nadel nach einem ersten Einstechen und Koagulieren aus dem von ihr erzeugten Kanal zurückgezogen, innerhalb des Katheters verdreht und dann erneut mit einer etwas abgewandelten Richtung in das Myokard eingestochen werden, so daß an derselben Stelle der Herzinnenwand mehrere zu durchblutende Kanäle erzeugt werden können, die etwa entgegengesetzt voneinander oder sternförmig zueinander angeordnet sind. So kann frühzeitig und schnell eine großflächige Durchblutung erzielt werden, selbst wenn einzelne der erzeugten Kanäle keine zusätzlichen Seitenäste ausbilden sollten.

Die Nadel oder dergleichen Koagulationswerkzeug kann als Schraubwendel mit insbesondere angespitztem Ende ausgebildet sein. Somit kann mit ihr ein entsprechend gewendelter, also relativ langer Kanal gebildet werden, obwohl nur eine relativ geringe Dicke der Herzwand zur Verfügung steht. Darüber hinaus ergibt sich dadurch auch eine Fixierung des Katheters, so daß eine Störung des Koagulationsvorganges aufgrund des Herzschlages besser vermieden werden kann.

Eine demgegenüber abgewandelte Ausgestaltung der Erfindung kann vorsehen, daß zusätzlich zu einer gerade oder gekrümmten Nadel oder dergleichen Koagulationswerkzeug eine dessen Austritt aus dem Katheter umschließende Schraubwendel zum Fixieren des Katheterendes an der Innenseite der Herzwand vorgesehen ist. In diesem Falle kann das distale Katheterende mit Hilfe der Schraubwendel festgelegt werden, so daß die Herzbewegungen sich nicht ungünstig auf den mit der eigentlichen Koagulationsnadel durchzuführenden Perforationsvorgang auswirken können.

Dabei ist es möglich, diese zusätzlich zu der Nadel oder dergleichen vorgesehene Haltewendel auch als zusätzliche Elektrode oder als zusätzliches Koagulationswerkzeug auszubilden und mit dem Hochfrequenzgenerator zu verbinden, so daß dann also jeder Anlegevorgang des Katheters zu mehreren Perforationen gleichzeitig führt, wobei aber außerdem der Katheter gut an der Innenseite der Herzwand fixiert ist.

Das Koagulationswerkzeug kann eine Hohlnadel oder Kanüle sein, in deren Innerem insbesondere eine Leitung zu einem an ihrer Spitze oder nahe ihrer Spitze angeordneten Temperatursensor verlaufen kann, um der Temperatursensor kann mit einer Temperaturanzeige und/oder der Steuerung oder Regelung des Hochfrequenzgenerators verbunden sein. Somit kann beim Koagulieren des jeweiligen Kanales die Temperatur überwacht und eine zu starke Temperaturerhöhung - eventuell automatisch - verhindert werden. Außerdem kann durch die Hohlnadel oder Kanüle ein Kontrastmittel eingebracht werden, um den Verlauf der Perforation zu kontrollieren. Insbesondere kann so die Dicke der Herzwand bei der Angringung des Kanales bestmöglich ausgenutzt werden, ohne die Herzwand vollständig zu durchstechen.

Eine andere oder zusätzliche Möglichkeit, die Eindringtiefe der Nadel oder des Werkzeuges in das Myokard zu kontrollieren, kann darin bestehen, daß an dem distalen Ende des Katheters im Bereich des Austrittes der Nadel ein Ultraschallgeber zur Messung der Dicke der Herzwand und der Eindringtiefe der Nadel oder dergleichen angeordnet ist.

Eine weitere Ausgestaltung der Erfindung kann darin bestehen, daß die Nadel oder Kanüle an ihrer Außenseite von ihrem Austritt aus dem distalen Ende des Katheters ausgehend bis nahe zu ihrer Spitze isoliert ist, so daß nur die Spitze selbst blank und als Koagulationsbereich ausgebildet ist. Dadurch wird erreicht, daß beim Einführen der Nadel bereits eine Koagulation durchgeführt werden kann und diese dann jeweils nur in dem Bereich bewirkt wird, in dem sich die Nadelspitze gerade befindet, das heißt der durch die Perforation geschaffene Kanal wird jeweils an jeder seiner Stellen ungefähr eine gleiche zeitlang der Hochfrequenz ausgesetzt. Es ergibt sich somit eine gleichmäßige Koagulation an der Innenwand des dadurch gebildeten künstlichen Kanales in der Herzwand.

Ein weiterer Vorteil der Ausgestaltung der Nadel als Hohlnadel oder Kanüle besteht noch darin, daß dann durch diese Nadel Flüssigkeitsproben entnommen oder Medikamente injiziert werden können.

Insgesamt ergibt sich eine Vorrichtung, mit der Perforationen in der Herzwand dauerhaft angebracht werden können, ohne daß der Brustkorb geöffnet werden muß. In überraschender Weise wird auf eine an sich bewährte Technik zurückgegriffen, nämlich die, einen an der Herzinnenwand anbringbaren Katheter vorzusehen, der mit einem entsprechenden distalen Arbeitsende in die Herzinnenwand eindringen kann, so daß dann durch die an sich auch bekannte Koagulation die Perforation zu einem dauerhaften und von innen her durchbluteten Kanal gemacht werden kann. Eine Nachblutung an der Herzaußenseite wird gleichzeitig wirkungsvoll vermieden. Da eine schwere Brustkorboperation vermieden wird, können auch die Herzen solcher Patienten mit Löchern und Kanälen zur Versorgung mit frischem Blut aus der Herzkammer versehen werden, die eine derartig schwere Operation nicht aushalten würden.

Nachstehend sind Ausführungsbeispiele der Erfindung anhand der Zeichnung näher beschrieben. Es zeigt in zum Teil schematisierter Darstellung:
- Fig.1: einen Katheter, der intrakardial in das Herz eines Patienten eingeführt ist und mit seinem Arbeitsende die Innenseite der Herzwand beaufschlagt, wobei das entgegengesetzte Ende an einen Hochfrequenzgenerator angeschlossen ist, an den außerdem eine mit dem Patienten in Berührung befindliche indifferente Elektrode verbunden ist,
in vergrößertem Maßstab:
- Fig.2: einen Längsschnitt durch das Herz, in welches das distale Ende des Katheters mit seinem Arbeitsende eingeführt ist,
in noch weiter vergrößertem Maßstab:
- Fig.3: das Arbeitsende des Katheters mit einer vorschiebbaren und zurückziehbaren, von der Innenseite her in die Herzwand eingestochenen, als Elektrode ausgebildeten Nadel zum Erzeugen einer Perforation in der Herzwand,
- Fig.4: eine der Fig.3 entsprechende Darstellung, bei welcher die Koagulationsnadel nahe ihrer Spitze einen Temperaturfühler aufweist,
- Fig.5: eine Ausführungsform, bei welcher die Nadel als Wendel ausgebildet ist,
- Fig.6: eine Ausführungsform, bei welcher eine gekrümmte und zurückziehbare Nadel und eine Schraubwendel am distalen Ende des Katheters angeordnet sind, wobei die gekrümmte Nadel etwa über einen Viertelkreis reicht,
- Fig.7: eine Ausführungsform, bei welcher am distalen Ende des Katheters ein Ultraschallsensor zur Ermittlung der Dicke der Herzwand und der Eindringtiefe der Koagulations-Nadel angeordnet ist,
- Fig.8: eine Ausführungsform, bei welcher die Koagulationsnadel über den größten Teil ihrer Länge isoliert ist, so daß nur der Bereich ihrer Spitze zum Koagulieren blank ausgebildet ist,
- Fig.9: eine Ausführungsform mit einer vorgekrümmten Nadel, die außerdem drehbar in dem Katheter angeordnet ist,
- Fig.10: eine Ausführungsform mit einer gekrümmten Nadel, die nahezu über einen Halbkreis reicht und so lang ist, daß sie mit ihrem freien Ende die Innenseite der Herzwand ein zweites Mal durchsticht sowie
- Fig.11: eine Ausführungsform mit einer vorgekrümmten Nadel, die zunächst aus dem Katheter mehr und mehr ausschiebbar ist, bis sie etwa über einen Halbkreis reicht, und dann im folgenden in gleicher Weise wieder zurückziehbar ist, wobeieinzelneZwischenpositionendieserAusschiebbarkeit und Rückziehbarkeit in Fig.11 untereinander dargestellt sind.

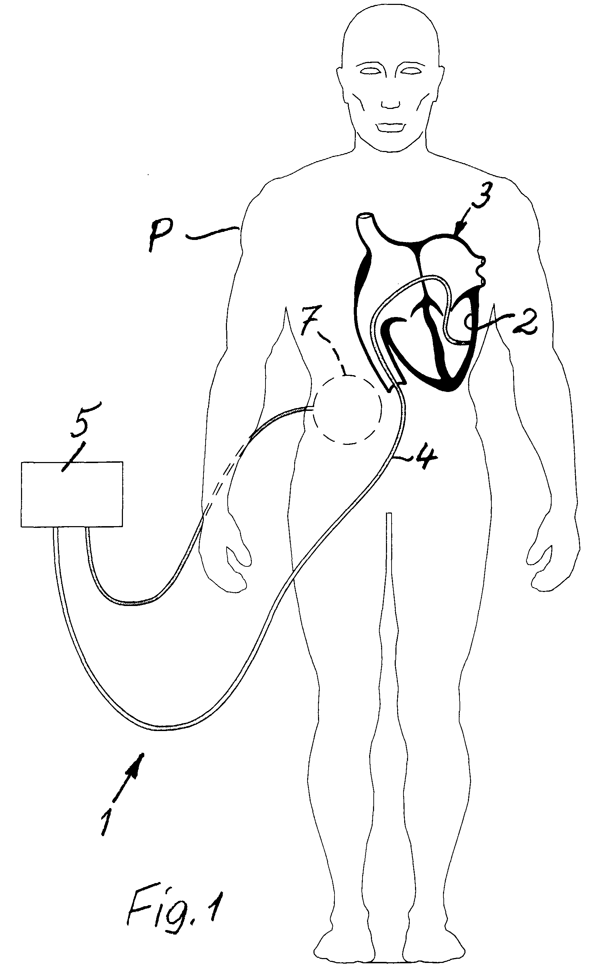

Eine in Fig.1 insgesamt schematisiert dargestellte, im ganzen mit 1 bezeichnete Vorrichtung dient zum Anbringen von Perforationen, Lochungen oder Kanälen in der Wand 2 eines Herzens 3, im folgenden auch Herzwand oder Myokard 2 genannt. Dadurch können in der Herzwand 2 verlaufende Kanäle erzeugt werden, durch welche die Herzwand mit frischem Blut aus dem Herzinneren versorgt werden kann, das heißt die Herzwand kann besser durchblutet werden, wenn derartige Kanäle mit der Vorrichtung 1 in noch zu beschreibender Weise angebracht werden.

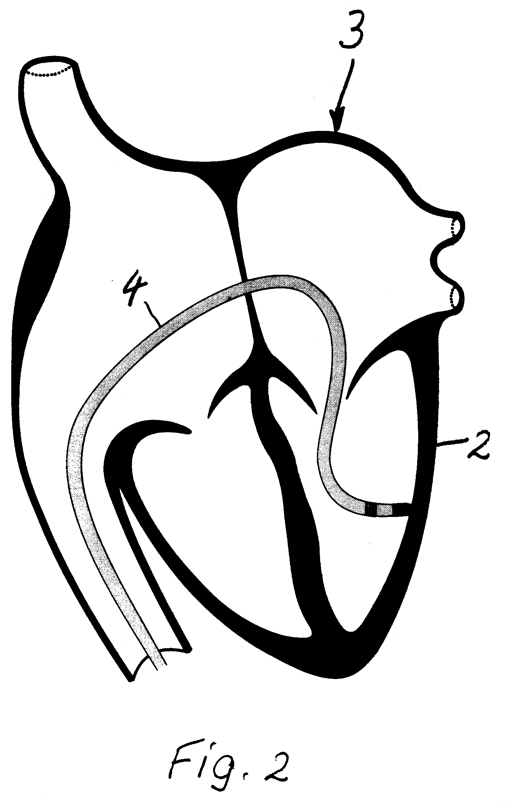

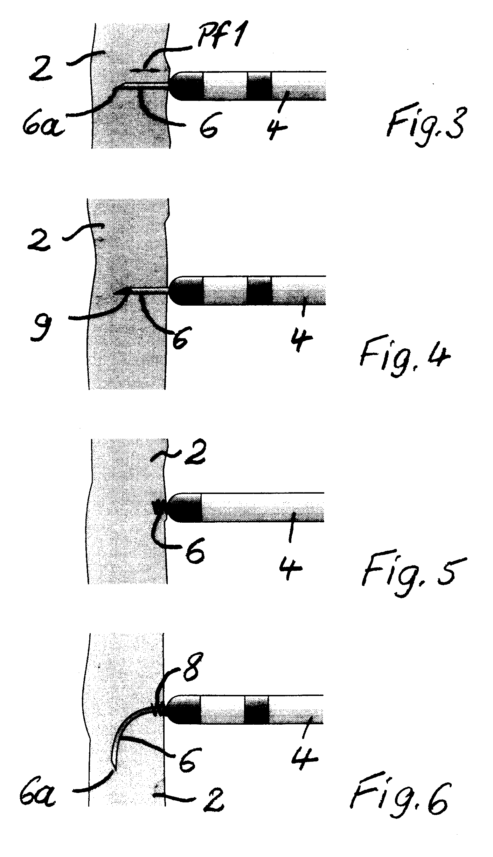

Die Vorrichtung 1 weist vor allem einen Katheter 4 mit einem Arbeitsende auf, das wenigstens eine Elektrode oder einen Pol und eine Verbindung dieser Elektrode oder des Poles zu einem Hochfrequenzgenerator 5 hat und gemäß Fig.1 und 2 intrakardial in das Herz 3 einführbar ist. Das erwähnte distale Arbeitsende weist gemäß den Figuren 3 bis 10 wenigstens eine von der Innenseite in die Herzwand 2 einstechbare gerade, gekrümmte und/oder gewendelte Nadel 6, also ein Perforations-Werkzeug, als Hochfrequenzpol oder - elektrode auf. Dieses Koagulationswerkzeug in Form einer Nadel 6 kann also nach dem Einführen des Katheters 4 in das Innere des Herzens 3 von der Innenseite her in die Herzwand 2 eingestochen werden, wie es in den Figuren 2 bis 10 angedeutet oder dargestellt ist, wobei oder wonach mit Hilfe des Hochfrequenzgenerators 5 eine Koagulation dieser Lochung oder Perforation durchgeführt wird, damit sie dauerhaft bleibt und nicht wieder verheilt. Somit kann in eine so gebildete Perforation von der Innenseite des Herzens 3 her frisches Blut eintreten und damit die Herzwand 2 an dieser Stelle durchbluten.

In den dargestellten Ausführungsbeispielen ist eine monopolare Koagulationsnadel 6 als Arbeitsende oder Koagulationswerkzeug vorgesehen und es ist gemäß Fig.1 mit dem Hochfrequenzgenerator 5 eine zweite, indifferente Elektrode 7 zur außenseitigen Anwendung an einem Patienten P verbunden.

Denkbar wäre auch eine bipolare Koagulationsnadel 6 als Arbeitsende, die zueinander beabstandet und gegeneinander isoliert beide mit dem Hochfrequenzgenerator 5 verbundenen oder verbindbaren Elektroden oder Pole aufweisen könnte.

In Fig.3 ist durch den Doppelpfeil Pf1 angedeutet - was auch für die übrigen Ausführungsbeispiele gilt - daß die Nadel 6 in das distale Ende des Katheters 4 einschiebbar oder einziehbar und zum Einstechen in die Innenseite der Herzwand 3 daraus ausschiebbar ist, wobei in allen Figuren die ausgeschobene Position dargestellt ist. Dabei erkennt man auch in allen Figuren, daß der Verschiebeweg der Nadel 6 relativ zu dem distalen Ende des Katheters 4 geringer als die Dicke der Herzwand 2 ist, wobei gemäß Fig.6, 9 und 10 die Nadel 6 auch elastisch und vorgekrümmt sein kann, so daß sie beim Auschieben aus dem distalen Ende des Katheters 4 in ihre gekrümmte Form übergeht und dann zwar insgesamt eine größere Länge haben kann, als es der Dicke der Herzwand 2 entspricht, wobei aber die in Fortsetzungsrichtung des Katheters 4 gemessene Länge oder Ausdehnung dieser Nadel wiederum geringer als die Dicke der Herzwand 2 ist, so daß in all diesen Fällen vermieden wird, daß die Herzwand 2 vollständig durchstochen wird. Somit wird ein Blutaustritt an der Außenseite des Herzens 3 vermieden.

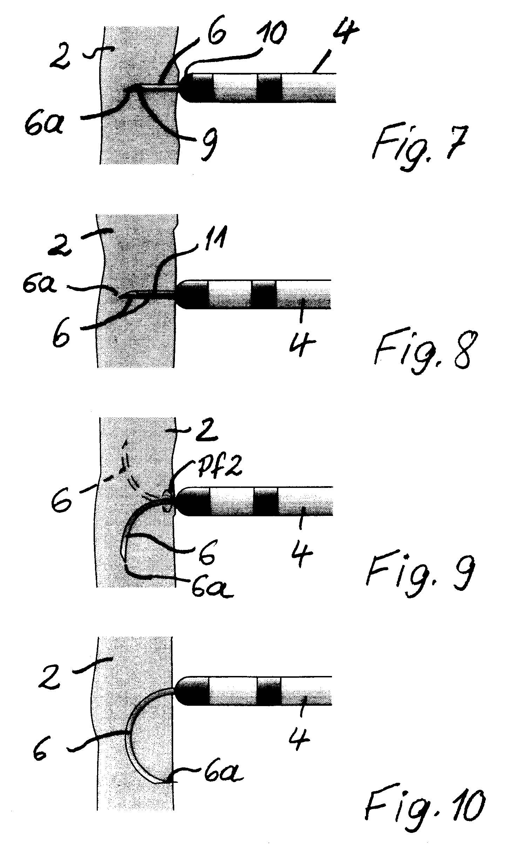

Gemäß Fig.9 kann die Nadel 6 oder dergleichen Koagulationswerkzeug relativ zu dem Katheter 4 verdrehbar sein, wobei die Drehlagerung und Drehbetätigung nicht näher dargestellt ist, weil sie mit bekannten Mitteln durchgeführt werden kann, wie man sie auch von anderen relativ zu einem Katheter verdrehbaren Arbeitsspitzen kennt. Dadurch ist es möglich, gemäß Fig.9 die Nadel 6 zunächst auszuschieben und einen in der dargestellten Figur sich gekrümmt nach unten erstreckenden Kanal in der Herzwand 2 anzubringen, anschließend die Nadel in den Katheter 4 zurückzuziehen, gemäß dem gekrümmten Pfeil Pf2 zu verdrehen und erneut bei gleichbleibender Lage des Katheters 4 in die Herzwand einzustechen, so daß sich mit einer übereinstimmenden Eintrittsöffnung zwei verzweigende Perforationen oder Kanäle ergeben. Die Vorkrümmung der Nadel 6 wird dabei aufgrund ihrer Elastizität beim Zurückziehen gegen die Federspannung aufgehoben, so daß die Nadel 6 innerhalb des Katheters 4 trotz dieser Krümmung Platz findet. Dabei kann die Länge und Form der Krümmung auch gemäß Fig.10 etwa bis zu einem Halbkreis gehen, so daß bei genügend weitem Ausschieben der Nadel 6 deren Spitze 6a wieder aus der Herzwand 2 zum Inneren des Herzens 3 austreten kann, um unter Umständen eine noch bessere Durchblutung zu erlauben.

Im Ausführungsbeispiel gemäß Fig.5 ist dargestellt, daß die Nadel 6 auch als Schraubwendel mit insbesondere angespitztem Ende ausgebildet sein kann. Sie erfüllt dann eine Doppelfunktion, indem sie beim Einführen in die Herzwand 2 die gewünschte, in diesem Falle wendelförmige Perforation erzeugt und während des Koagulationsvorganges den Katheter 4 und sich selbst festlegt, so daß Herzbewegungen nicht zu ungewollten Verschiebungen oder gar einem Wiederaustritt der Nadel 6 aus der Herzwand 2 führen kann.

Zwar kann eine vorgekrümmte, beim Ausschieben aus dem Katheter 4 und Einstechen in die Herzwand 2 ihre gekrümmte Position einnehmende Nadel 6 aufgrund dieser Formgebung auch eine gewisse Fixierung erzeugen, jedoch ist es außerdem möglich, zusätzlich zu einer geraden oder gekrümmten Nadel 6 eine deren Austritt aus dem Katheter 4 umschließende Schraubwendel 8 zum Fixieren des Katheterendes an der Innenseite der Herzwand 2 vorzusehen, wie es in Fig.6 dargestellt ist. Somit kann der Katheter 4 mit Hilfe der Schraubwendel 8 fixiert werden, während die in diesem Falle gekrümmte Nadel 6 in die Herzwand 2 eingestochen ist oder wird und für den Zeitraum der Koagulation liegenbleiben soll.

Dabei kann diese zusätzlich zu der Nadel 6 vorgesehene Haltewendel 8 gegebenenfalls selbst als zusätzliche Elektrode und als zusätzliches Koagulationswerkzeug ausgebildet und mit dem Hochfrequenzgenerator 5 verbindbar oder verbunden sein. Es ergibt sich dann praktisch eine Kombination der Ausführungsformen gemäß Fig.5 und 9, wobei aber auch eine gerade verlaufende Nadel 6 mit einer solchen Haltewendel 8 kombiniert sein kann.

Die Koagulationsnadel 6 kann eine Hohlnadel oder Kanüle sein, so daß damit auch Kontrastmittel zur Überprüfung des erzeugten, zur Durchblutung dienenden Kanales möglich ist oder es können Medikamente eingespritzt werden oder Probeflüssigkeit entnommen werden. In Fig.7 ist angedeutet, daß nahe der Spitze 6a einer solchen Nadel 6 ein Temperatursensor 9 angeordnet sein kann, wobei dann im Inneren der hohlen Nadel 6 eine Leitung von diesem Temperatursensor 9 zu einer Temperaturanzeige und/oder zu der Steuerung oder Regelung des Hochfrequenzgenerators 5 verlaufen kann, so daß der Koagulationsvorgang und insbesondere die dabei auftretende Temperatur überwacht und gegebenenfalls automatisch gesteuert oder geregelt werden kann. Somit können zu geringe oder zu hohe Temperaturen vermieden werden.

In Fig.7 ist außerdem angedeutet, daß an dem distalen Ende des Katheters 4 im Bereich des Austrittes der Nadel 6 ein Ultraschallgeber 10 zur Messung der Dicke der Herzwand 2 und der Eindringtiefe der Nadel 6 angeordnet sein kann, wobei dies auch bei den anderen Ausführungsbeispielen verwirklicht sein könnte.

Fig.8 zeigt ein Ausführungsbeispiel, bei welchem die Nadel 6 an ihrer Außenseite von ihrem Austritt aus dem distalen Ende des Katheters 4 ausgehend bis nahe zu ihrer Spitze 6a mit einer Isolierung 11 umhüllt ist, so daß nur die Spitze 6a selbst blank und als Koagulationsbereich ausgebildet ist. Somit kann der Koagulationsvorgang schon während des Einführens der Nadel 6 durchgeführt werden und entsprechend dem Vorschub der Nadel 6 fortschreiten. Durch die Wahl der Vorschubgeschwindigkeit kann Einfluß auf die Intensität der Koagulation genommen werden.

Es können also auf einfache Weise ohne Anwendung einer Thorakotomie für die Revaskularisation notwendige Kanäle im Myokard 2 des Herzens 3 erzeugt werden, wobei in vorteilhafter Weise ein steuerbarer, intrakardial oder transvenös einführbarer Katheter 4 vom Inneren des Herzens 3 aus solche Kanäle bis zu einer bestimmten Tiefe und ohne vollständiges Durchdringen der Herzwand 2 erzeugt. Dabei kann in üblicher Weise der Katheter 4 zunächst im Herzen 3 positioniert werden, wonach aus seinem Arbeitsende die entsprechende Nadel 6, Kanüle oder dergleichen Koagulationswerkzeug ausgefahren und in kontrollierte Tiefen des Myokards eindringen kann. Die Positionierung des Arbeitsendes des Katheters 4 kann durch Messung des intrakardialen EKGs vorgenommen werden. Danach erfolgt über die Nadel 6 gegen eine großflächige indifferente Elektrode 7, die zum Beispiel am Rücken des Patienten P angebracht werden kann, eine Stromabgabe mittels des Hochfrequenzgenerators 5, was zur Folge hat, daß der durch die Nadel 6 erzeugte Kanal teilweise koaguliert wird und damit sein Lumen im Myokard 2 offen bleibt. Da mit Hilfe einer gebogenen Nadel 6 auch entsprechend lange und gekrümmte Kanäle oder Perfusionslöcher erzeugt werden können, ergeben sich entsprechend längere "künstliche" Gefäße für eine Durchblutung des Myokards. Darüber hinaus kann aufgrund der Verbindung mit dem Hochfrequenzgenerator 5 die Vorrichtung 1 auch zur Hochfrequenzablation verwendet werden.

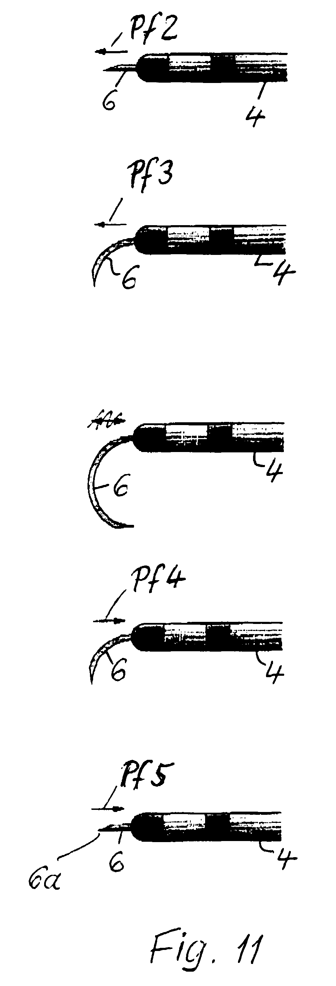

In Fig.11 ist die schon erwähnte Ausschiebbarkeit und Rückziehbarkeit der Nadel 6 in einzelnen Schritten noch deutlicher dargestellt. Dabei handelt es sich in diesem Falle um eine gekrümmte Nadel 6 etwa gemäß Fig.10. Diese wird gemäß dem Pfeil Pf2 zunächst aus dem Katheter vorgeschoben, wobei eine weitere Verschiebung gemäß dem Pfeil Pf3 dazu führt, daß die Nadel 6 ihre gekrümmte Lage einnimmt. Würde der Katheter 1 in dieser Position verwendet, hätte man praktisch eine etwa um einen Viertelkreis gekrümmte Nadel.

Das weitere Ausschieben führt schließlich dazu, daß die Nadel 6 über den ihr vorgegebenen Halbkreis reicht, wie er in Fig.10 dargestellt ist. Diese in Fig.11 mittlere Darstellung entspricht also der Benutzungsform. Die zuvor erreichte Position könnte hingegen einer Benutzung gemäß Fig.9 entsprechend.

Aus dieser Position kann die Nadel dann gemäß dem Pfeil Pf4 zunächst teilweise und gemäß dem Pfeil Pf5 dann wieder ganz zurückgezogen werden.

Dabei kann die Zurückziehung so weit gehen, daß die Nadel 6 vollständig in dem Katheter 4 verschwindet. Es kann aber noch ein Stück und insbesondere die Spitze 6a überstehen, falls dies erwünscht ist, um den Arbeitsvorgang des Einstechens zu erleichtern. Dieser Vorgang gemäß Fig.11 kann gegebenenfalls mehrfach wiederholt werden, um entsprechend viele Herzkanäle von der Innenseite des Herzens her anzubringen.

Die Vorrichtung 1 dient zum Anbringen von Perforationen, Lochungen oder Perfusionskanälen in der Herzwand 2, insbesondere im Myokard, zur Erzeugung von in der Herzwand 2 verlaufenden bleibenden Kanälen für deren Durchblutung. Damit der Brustkorb zum Anbringen solcher Perforationen nicht in einer Operation geöffnet werden muß, um das Herz freizulegen, weist die Vorrichtung 1 einen intrakardial in das Herz 3 einführbaren Katheter 4 auf, der an seinem Arbeitsende wenigstens eine Elektrode oder einen Pol und eine Verbindung dieser Elektrode oder des Poles zu einem Hochfrequenzgenerator 5 hat. Dieses distale Arbeitsende ist dabei wenigstens eine von der Innenseite in die Herzwand 2 einstechbare Nadel 6, die als Koagulationswerkzeug wirkt, so daß das Lumen des von ihr erzeugten Kanales offen bleibt und von dem Inneren des Herzens 3 aus mit frischem Blut versorgt werden kann.

## Patentansprüche

1. Vorrichtung (1) zum Anbringen von Perforationen, Lochungen oder Kanälen in der Herzwand (2), insbesondere im Myokard, zur Erzeugung von in der Herzwand (2) verlaufenden Kanälen für deren Durchblutung, **dadurch gekennzeichnet**, daß die Vorrichtung (1) einen Katheter (4) mit einem Arbeitsende aufweist, das wenigstens eine Elektrode oder einen Pol und eine Verbindung dieser Elektrode oder des Poles zu einem Hochfrequenzgenerator (5) hat und intrakardial in das Herz (3) einführbar ist, und daß dieses distale Arbeitsende wenigstens eine von der Innenseite in die Herzwand (2) mehrfach einstechbare gerade, gekrümmte und/oder gewendelte Nadel (6) oder Werkzeug als Hochfrequenzpol oder -elektrode aufweist.

2. Vorrichtung nach Anspruch 1, dadurch gekennzeichnet, daß eine monopolare Koagulationsnadel (6) als Arbeitsende oder Werkzeug vorgesehen ist und eine zweite, indifferente Elektrode (7) zur außenseitigen Anwendung an einem Patienten (P) mit dem Hochfrequenzgenerator (5) verbindbar ist.

3. Vorrichtung nach Anspruch 1, dadurch gekennzeichnet, daß eine bipolare Koagulations-Nadel (6) als Arbeitsende vorgesehen ist, die zueinander beabstandet und gegeneinander isoliert beide mit dem Hochfrequenzgenerator (5) verbundenen oder verbindbaren Elektroden oder Pole aufweist.

4. Vorrichtung nach einem der Ansprüche 1 bis 3, dadurch gekennzeichnet, daß die Nadel (6) oder das Werkzeug in das distale Ende des Katheters (4) einschiebbar oder einziehbar und zum Einstechen in die Innenseite der Herzwand (3) daraus ausschiebbar ist.

5. Vorrichtung nach einem der Ansprüche 1 bis 4, dadurch gekennzeichnet, daß die gekrümmte Nadel (6) etwa einen Viertelkreis bis einen Halbkreis oder gegebenenfalls etwas mehr als einen Halbkreis bildet.

6. Vorrichtung nach einem der Ansprüche 1 bis 5, dadurch gekennzeichnet, daß der Verschiebeweg der Nadel (6) oder des Werkzeuges relativ zu dem distalen Ende des Katheters (4) geringer als die Dicke der Herzwand (2) ist.

7. Vorrichtung nach einem der Ansprüche 1 bis 6, dadurch gekennzeichnet, daß die Nadel (6) elastisch und vorgekrümmt ist und beim Ausschieben aus dem distalen Ende des Katheters (4) in ihre gekrümmte Form übergeht.

8. Vorrichtung nach einem der Ansprüche 1 bis 7, dadurch gekennzeichnet, daß die Koagulationsnadel (6) oder dergleichen Werkzeug relativ zu dem Katheter (4) verdrehbar ist.

9. Vorrichtung nach einem der Ansprüche 1 bis 8, dadurch gekennzeichnet, daß die Nadel (6) oder dergleichen Koagulations-Werkzeug als Schraubwendel mit insbesondere angespitztem Ende ausgebildet ist.

10. Vorrichtung nach einem der Ansprüche 1 bis 9, dadurch gekennzeichnet, daß zusätzlich zu einer geraden oder gekrümmten Nadel (6) oder dergleichen Koagulations-Werkzeug eine deren Austritt aus dem Katheter (4) umschließende Schraubwendel (8) zum Fixieren des Katheterendes an der Innenseite der Herzwand (2) vorgesehen ist.

11. Vorrichtung nach einem der Ansprüche 1 bis 10, dadurch gekennzeichnet, daß die zusätzlich zu der Nadel (6) oder dergleichen vorgesehene Haltewendel (8) als zusätzliche Elektrode und als zusätzliches Koagulationswerkzeug ausgebildet und mit dem Hochfrequenzgenerator (5) verbindbar ist.

12. Vorrichtung nach einem der Ansprüche 1 bis 11, dadurch gekennzeichnet, daß das Koagulationswerkzeug eine Hohlnadel oder Kanüle ist, in deren Innerem insbesondere eine Leitung zu einem an ihrer Spitze oder nahe ihrer Spitze angeordneten Temperatursensor verläuft, und daß der Temperatursensor mit einer Temperaturanzeige und/oder der Steuerung oder Regelung des Hochfrequenzgenerators (5) verbunden ist.

13. Vorrichtung nach einem der Ansprüche 1 bis 12, dadurch gekennzeichnet, daß an dem distalen Ende des Katheters (4) im Bereich des Austrittes der Nadel (6) ein Ultraschallgeber (10) zur Messung der Dicke der Herzwand (2) und der Eindringtiefe der Nadel (6) oder dergleichen angeordnet ist.

14. Vorrichtung nach einem der Ansprüche 1 bis 13, dadurch gekennzeichnet, daß die Nadel (6) oder Kanüle an ihrer Außenseite von ihrem Austritt aus dem distalen Ende des Katheters (4) ausgehend bis nahe zu ihrer Spitze (6a) isoliert ist, so daß nur die Spitze (6a) selbst blank und als Koagulationsbereich ausgebildet ist.
